# EUROPÄISCHE PATENTSCHRIFT

(11) **EP 1 413 270 B1**
(45) Veröffentlichungstag und Bekanntmachung des Hinweises auf die Patenterteilung: **15.07.2009**
(21) Anmeldenummer: 03024321.6
(22) Anmeldetag: 24.10.2003
(51) Int. Cl.: A61F 13/00, A61K 9/70, A61L 15/44

(54) **Wundkontaktmaterial**
Wound dressing
Pansement pour plaie

(30) Priorität: 24.10.2002 DE 10250848
(43) Veröffentlichungstag der Anmeldung: 28.04.2004
(73) Patentinhaber: Paul Hartmann Aktiengesellschaft, 89522 Heidenheim (DE)
(72) Erfinder: Hierlemann, Helmut, Dr., 73033 Göppingen (DE); Coerper, Stephan, Dr. med., 72070 Tübingen (DE); Planck, Heinrich, Dr. med., 72622 Nürtingen (DE); Doser, Michael, Dr., 70794 Filderstadt (DE); Hanano, Ralph, Dr., 89359 Koetz (DE)
(74) Vertreter: Patentanwälte Ruff, Wilhelm, Beier, Dauster & Partner

(56) Entgegenhaltungen:
- EP-A- 0 170 821
- EP-A- 0 353 972
- WO-A-02/102358
- DE-A- 3 202 775

## Beschreibung

Die Erfindung betrifft ein Wundkontaktmaterial, insbesondere zur Behandlung von schlecht heilenden und chronischen Wunden wie Ulcera, mit einem flächigen Trägermaterial, welches wundheilungsförderndes freisetzbares Material aufweist, das ungleichmäßig über die Fläche des Trägermaterials verteilt ist.

Chronische, beispielsweise durch Diabetes mellitus, chronisch-venöse Insuffizienz entstandene Wunden oder Decubiti heilen auch nach Ausschaltung systemischer Störfaktoren durch Revaskularisation, Stoffwechseleinstellung und adäquate Lokaltherapie in mehr als der Hälfte aller Fälle nicht ab oder rezidivieren frühzeitig. Die komplexe Interaktion der an der Gewebsregeneration beteiligten lokalen und systemischen Zellen unterliegt im Rahmen einer komplexen Wundheilungskaskade einer Steuerung durch Wachstumsfaktoren. Ihre chemotaktischen, proliferativen und synthetischen Eigenschaften werden endokrin, autokrin und parakrin vermittelt. Während in der Initialphase nach Gewebstrauma Thrombozyten, Leukozyten, Lymphozyten und Makrophagen die inflammatorische und exsudative Frühphase der Wundheilung bestimmen, so sind es in späteren, auf Proliferation ausgerichteten Abschnitten die ortsständigen Zellen des Reparationsgewebes, wie Endothelzellen, Fibroblasten und Keratinozyten, welche zur autokrinen und parakrinen Produktion von Wachstumsfaktoren befähigt sind. Übereinstimmend zeigen tierexperimentelle Daten, dass durch lokale und systemische Gabe von wundheilungsfördernden Substanzen wie Wachstumsfaktoren, eine Modulation der Gewebsreparation erzielt werden kann (Coerper S, Schäffer M, Enderle M, Schott U, Köveker G, Becker HD: Die chirurgische Wundsprechstunde: Ein interdisziplinäres Zentrum zur Behandlung chronischer Wunden durch standardisierte und kontrollierte Therapie. Chirurg 1999; 4:480-484). Auch erste klinische Humanstudien belegen die modulatorische Wirkung von exogen applizierten Wachstumsfaktoren auf die Wundheilung (Wiemann TJ: Clinical efficacy of beclaplermin (rhPDGF-BB) gel. Am J Surg 1999; 176:74S-79S).

Schlecht heilende Wunden wie beispielsweise Ulcera sind meist durch eine starke Produktion von Wundexsudat gekennzeichnet. Für einen raschen Wundheilungsfortschritt ist die Schaffung eines geeigneten Wundmilieus von Nöten. Die Optimierung des Wundmilieus als richtige Einflussgröße der Wundheilung geschieht am besten durch eine Verbandstechnik, welche die Wunde feucht hält. Die Kompatibilität der feuchten Verbandstechnik mit der aktiven pharmakologischen Stimulation von Wunden ist mit den derzeitigen Materialien jedoch nicht gegeben. Einerseits sind Dampfdurchlässigkeit, Nonadhäsivität und Schutz vor bakterieller Kontamination wichtige Charakteristika von Wundverbänden. Andererseits müssen die für die aktive Gewebsstimulation vorgesehenen Verbände die Ankopplung von wundheilungsfördernden Materialien wie Wachstumsfaktoren und ihre bioaktive Abgabe an die Wundumgebung ermöglichen.

Aus dem Stand der Technik (EP 0 808 158 B1) sind Wundkontaktmaterialien bekannt, welche wundheilungsfördernde Substanzen enthalten und an die wunde Körperstelle abgeben.

Die WO 02/102358 A1 beschreibt ein Wundkontaktmaterial mit einer vollflächigen Beschichtung mit wundheilungsfördenden Mitteln.

Die DE 32 02 775 A1 betrifft ein Drug Delivery, bei dem es darum geht, bestimmte Medikamente durch die gesunde Haut hindurch zu transportieren. Der Wirkstoff, der durch die Haut hindurch transportiert werden soll, ist stellenweise aufgetragen.

In EP 0 353 972 A1 befasst sich eine Ausführung wiederum mit einem Drug Delivery. Für den Hautkontakt können neben üblichen Klebstoffpunkten noch zusätzliche Klebstoffpunkte vorhanden sein, die mit den durch die Haut zu transportierenden Stoffen versehen sein können.

Die EP 0 170 821 A1 zeigt ebenfalls einen transdermalen Applikator, d.h. ein Drug Delivery. Eine Trägerbahn, die in Form einer Folie vorliegen kann, wird vollflächig mit einem Klebstoff beschichtet. Auf die Klebstoffschicht werden die zur transdermalen Applikation bestimmten Wirkstoffe aufgetragen, wobei zwischen der Klebstoffschicht und den Wirkstoffen Trennfolienstücke vorgesehen sind, um eine Interaktion zwischen der Klebstoffmasse und den Wirkstoffen zu vermeiden.

Die Aufgabe der vorliegenden Erfindung ist es, ein Wundkontaktmaterial zu schaffen, welches gegenüber Wundkontaktmaterialien aus dem Stand der Technik verbesserte Eigenschaften, insbesondere hinsichtlich der Wundheilungsgeschwindigkeit von Wunden, insbesondere von schlechtheilenden Wunden wie Ulcera, aufweist.

Diese Aufgabe wird durch ein Wundkontaktmaterial der eingangs genannten Art erfindungsgemäß dadurch gelöst, dass das Trägermaterial hinsichtlich der Durchlässigkeit von Wundexsudat inhomogen aufgebaut ist und Stellen hoher und Stellen niedriger Durchlässigkeit für Wundexsudat aufweist, wobei das wundheilungsfördernde Material im Bereich der Stellen mit geringer Durchlässigkeit für Wundexsudat vorgesehen ist. Als wundheilungsförderndes Material kommen insbesondere Wachstumsfaktoren und/oder Zellen in Frage.

Durch diese erfindungsgemäße ungleichmäßige, insbesondere diskontinuierliche Verteilung des wundheilungsfördernden Materials kommt es zu einer topographisch unterschiedlichen Abgabe des wundheilungsfördernden Materials an die Wunde Dies bewirkt eine lokal konzentrierte Abgabe des genannten wundheilungsfördernden Materials an die Wunde. Wird das Wundkontaktmaterial häufig gewechselt (vorzugsweise wird das Wundkontaktmaterial täglich gewechselt), so werden auch ständig unterschiedliche Wundstellen mit unterschiedlich hohen Konzentrationen von wundheilungsförderndem Material versorgt. So werden bei jedem Materialwechsel andere Wundstellen zur Heilung stimuliert. Die ungleichmäßige Verteilung des wundheilungsfördernden Materials kann von einem allmählichen Übergang von höher konzentrierten Stellen zu weniger konzentrierten Stellen bestehen. Vorzugsweise liegt ein abrupter Übergang von Stellen mit wundheilungsförderndem Material und Stellen ohne wundheilungsförderndem Material vor. Die Flächenverteilung kann zwischen 1 zu 4 und 4 zu 1 liegen. In der Regel haben die Stellen mit wundheilungsförderndem Material bzw. erhöhter Konzentration eine Mindestgröße von 1mm². Diese Stellen können voneinander getrennt oder auch miteinander verbunden sein.

Die erfindungsgemäße ungleichmäßige Verteilung des wundheilungsfördernden Materials kann beispielsweise so erreicht werden, dass das wundheilungsfördernde Material punkt- oder streifenförmig auf das Trägermaterial aufgetragen wird. Gemäß der Erfindung wird die inhomogene Verteilung des wundheilungsfördernden Materials in Verbindung mit einer inhomogenen Durchlässigkeit des Trägermaterials für Wundexsudat verwirklicht. So ist beim erfindungsgemäßen Wundkontaktmaterial das Trägermaterial hinsichtlich der Durchlässigkeit von Wundexsudat über die Fläche des Trägermaterials inhomogen aufgebaut. Dies bedeutet, dass das Trägermaterial Stellen unterschiedlicher Durchlässigkeit für Wundexsudat aufweist.

Durch die Tatsache, dass das Trägermaterial hinsichtlich der Durchlässigkeit von Wundexsudat inhomogen aufgebaut ist und Stellen hoher und Stellen niedriger Durchlässigkeit für Wundexsudat aufweist, wird erreicht, dass überschüssiges Wundexsudat aus der Wunde austreten kann. Das wundheilungsfördernde Material ist im Bereich der Stellen mit niedriger Durchlässigkeit für Exsudat vorgesehen. Durch die inhomogene Struktur des Trägermaterials wird ein gesteuerter Abfluss des Wundexsudats ermöglicht, wobei ein vorzeitiges Ausschwemmen von wundheilungsförderndem Material durch das Exsudat, wie dies bei homogenen Strukturen des Trägermaterials der Fall ist, verhindert wird.

Das Trägermaterial kann beispielsweise unterschiedliche Materialstärken aufweisen. An Stellen, an denen das Trägermaterial dünner ist, ist dann die Durchlässigkeit für Wundexsudat höher als an Stellen höherer Materialstärke. Denkbar ist auch ein Wundkontaktmaterial, welches ein Trägermaterial, bestehend aus mindestens zwei verschiedenen Materialien unterschiedlicher Durchlässigkeit für Wundexsudat, umfasst.

Bei einer bevorzugten Variante des erfindungsgemäßen Wundkontaktmaterials weist das Trägermaterial Durchbrechungen bzw. Materialaussparungen auf. Das Trägermaterial kann beispielsweise gelocht oder geschlitzt sein. Die Durchbrechungen bzw. Aussparungen können hinsichtlich ihrer Größe und/oder Verteilung auf der Gesamtfläche des Wundkontaktmaterials gleichmäßig oder ungleichmäßig verteilt sein. Durch diese Durchbrechungen bzw. Materialaussparungen kann das Wundexsudat von der Wunde weg nach außen strömen, ohne dass wundheilungsförderndes Material in größeren Mengen mit ausgeschwemmt wird. Das wundheilungsfördernde Material wiederum kann im Gegenzug erleichtert in die Wunde hinein diffundieren.

Durch die beschriebene inhomogene Struktur des Trägermaterials, beispielsweise durch Materialaussparungen, wird nicht nur ein erleichterter Austritt von Wundexsudat ermöglicht, sondern es wird gleichzeitig die ebenfalls oben genannte ungleichmäßige Verteilung wundheilungsfördernden Materials erreicht. Dies stellt einen besonderen Vorteil des erfindungsgemäßen Wundkontaktmaterials dar.

Mit Vorteil ist das erfindungsgemäße Wundkontaktmaterial im wesentlichen flexibel ausgebildet. Dadurch ist es an die verschiedenen anatomischen Gegebenheiten anpassbar. So ist es nicht nur an flächigen Körperpartien wie beispielsweise am Rücken, sondern auch an Extremitäten oder gar Fingern anpassbar. Vorzugsweise ist es elastisch und/oder plastisch verformbar ausgebildet.

Das Trägermaterial kann mit dem wundheilungsfördernden Material beschichtet sein. Hierzu kann beispielsweise das fertige Trägermaterial in eine Lösung mit wundheilungsförderndem Material eingetaucht oder mit einer solchen besprüht und anschließend getrocknet werden. Auch eine Bedruckung des Trägermaterials mit wundheilungsförderndem Material ist vorteilhaft. Damit kann in beliebiger Weise eine topographische Inhomogenität erreicht werden.

Bei einer weiteren vorteilhaften Variante des erfindungsgemäßen Wundkontaktmaterials ist das wundheilungsfördernde Material in das Trägermaterial inkorporiert, d.h. das Trägermaterial ist mit dem wundheilungsfördernden Material durchsetzt. Ein solches Wundkontaktmaterial kann beispielsweise so hergestellt werden, dass das Trägermaterial sowie das wundheilungsfördernde Material in flüssigem Zustand miteinander vermischt werden und anschließend in die gewünschte Form gebracht werden. Danach kann das flächig geformte Wundkontaktmaterial getrocknet werden.

Vorzugsweise ist das flächige Trägermaterial als Folie, poröse Membran, Vlies, Gewebe, Gestrick, Gewirk oder Geflecht oder Kombinationen davon ausgebildet. Mit Vorteil besitzt das erfindungsgemäße Wundkontaktmaterial eine Dicke von 20 bis 200 µm. Besonders bevorzugt ist das Wundkontaktmaterial als Folie, vorzugsweise mit einer Dicke von 20 bis 80 µm, insbesondere 30 bis 70 µm, ausgebildet. Vorzugsweise wird eine solche Folie bei ihrer Herstellung aus einer Lösung luftgetrocknet. Durchbrechungen können schon beim Trocknen ausgebildet oder nachträglich vorgesehen werden.

Bei einer weiteren bevorzugten Variante des erfindungsgemäßen Wundkontaktmaterials ist dieses als poröse Membran mit einer Dicke von 40 bis 200 µm, insbesondere 75 bis 150 µm, ausgebildet. Vorzugsweise wird die genannte poröse Membran zu ihrer Herstellung gefriergetrocknet, wobei die Poren gebildet werden. Bei einer porösen Membran können die Poren alleine als Austrittsöffnungen für Wundexsudat und für eine ungleichmäßige Verteilung von wundheilungsförderndem Material dienen. Bevorzugt ist es jedoch, dass die Inhomogenität der Membran zusätzlich, beispielsweise durch Materialaussparungen, erhöht ist.

Besonders bevorzugt ist es, dass das Trägermaterial aus quellfähigem und/oder wasseraufnahmefähigem Material besteht. Bevorzugt besteht das Trägermaterial aus mindestens einem Stoff aus der Gruppe bestehend aus Polyurethan, Polyvinylalkohol, Polyethylenglycol, Silikon, Cellulose, Carboxymethylcellulose, Alginat, Polysaccharid, Chitin, Chitosan und Polyvinylpyrrolidon. Besonders bevorzugt ist das Wundkontaktmaterial als perforierte Folie ausgebildet. Das Trägermaterial besteht vorzugsweise aus einem Gemisch aus Polyvinylalkohol und Polyethylenglycol.

Mit Vorteil umfasst das wundheilungsfördernde Material mindestens einen Wirkstoff pharmakologischen Ursprungs. Vorteilhaft als wundheilungsfördernde Materialien sind Kortikoide, Analgetika, Vitamine, Antibiotika, antivirale Stoffe oder Mischungen davon. Ebenfalls, je nach Wundbild, eignen sich Proteine, insbesondere Zytokine, als wundheilungsfördernde Materialien.

Wie bereits eingangs erwähnt, spielen Wachstumsfaktoren eine wichtige Rolle bei der Wundheilung. Übereinstimmend zeigen tierexperimentelle Daten, dass durch lokale und systemische Gabe von Wachstumsfaktoren eine Modulation der Gewebsreparation erzielt werden kann. Deshalb umfasst das wundheilungsfördernde Material besonders bevorzugt Wachstumsfaktoren, insbesondere PDGF, IGF, EGF, TGF, FGF, oder Mischungen davon. Insbesondere Wachstumsfaktoren wie PDGF, TGFbeta, FGF und IGF-1 spielen eine besondere Rolle für die Gewebsreparation. Auch erste klinische Humanstudien belegen die modulatorische Wirkung von exogen applizierten Wachstumsfaktoren auf die Wundheilung.

Bei einer weiteren vorteilhaften Variante des erfindungsgemäßen Wundkontaktmaterials umfasst das wundheilungsfördernde Material menschliche und/oder tierische Zellen, insbesondere Keratinozyten, Fibroblasten, Chondrozyten, Osteoblasten oder Kombinationen davon. Besonders bevorzugt weist das Wundkontaktmaterial eine Zelldichte von etwa 20.000 bis etwa 500.000 Zellen pro cm², vorzugsweise etwa 40.000 bis 150.000 Zellen pro cm² auf.

Mit Vorteil enthält das erfindungsgemäße Wundkontaktmaterial weniger als 100 µg Wirkstoff pro cm², vorzugsweise ca. 2 bis ca. 20 µg/cm², insbesondere ca. 5 µg/cm², Wirkstoff, bezogen auf die Gesamtfläche des Wundkontaktmaterials einschließlich der Flächen der Poren und/oder Durchbrechungen.

Bei einer bevorzugten Variante des erfindungsgemäßen Wundkontaktmaterials ist das Trägermaterial feuchtigkeits- und flüssigkeitsdurchlässig und ist mit einer Decklage, insbesondere zur Aufnahme von Wundflüssigkeit und zur Gewährleistung eines feuchten Wundmilieus, kombiniert. Besonders bevorzugt ist die Decklage mit dem Trägermaterial auf dessen wundabgewandter Seite verbunden. Mit Vorteil wird die Decklage von einem Flüssigkeit aufnehmbaren Material, besonders hervorzuheben sind hier feste Hydrogele oder Hydrokolloide, gebildet. Im Falle eines von Natur aus klebrigen Hydrogels kann das Trägermaterial mit diesem durch bloßes Andrücken verbunden werden. Es ist auch denkbar, dass das Trägermaterial mit der Decklage beispielsweise durch ein zusätzliches Befestigungsmittel wie Klebstreifen verbunden ist.

Die Erfindung betrifft auch die Verwendung des erfindungsgemäßen Wundkontaktmaterials zur Behandlung von schlecht heilenden Wunden, insbesondere von Hautwunden. Insbesondere wird das erfindungsgemäße Wundkontaktmaterial zur Behandlung von Ulcera verwendet.

Weitere Einzelheiten und Merkmale der Erfindung ergeben sich aus der nachfolgenden Beschreibung der Herstellung von erfindungsgemäßen Wundkontaktmaterialien und von bevorzugten Ausführungsformen in Verbindung mit den Unteransprüchen. Hierbei können die jeweiligen Merkmale jeweils für sich allein oder zu mehreren in Kombination miteinander verwirklicht sein.

In den Zeichnungen zeigen:
- Figur 1:: eine Aufsicht auf eine Ausführungsform mit homogener Verteilung von quadratischen Aussparungen.
- Figur 2:: eine Aufsicht auf eine Ausführungsform mit homogener Verteilung von rechteckigen Aussparungen.
- Figur 3:: eine Aufsicht auf eine Ausführungsform mit inhomogener Verteilung und unterschiedlichen Größen von quadratischen und rechteckigen Aussparungen.
- Figur 4:: Freisetzungskinetik von IGF aus einem erfindungsgemäßen Wundkontaktmaterial in vitro (Langzeit-Graph).
- Figur 5:: Vergleich der Reduktion der Wundgröße in % bei Verwendung eines Hydrogelverbands und eines erfindungsgemäßen Wundkontaktmaterials, dotiert mit dem wundheilungsförderndem Wachstumsfaktor IGF-1 (Beladung 5µg/cm²) bei normal und schlecht heilenden Wunden.

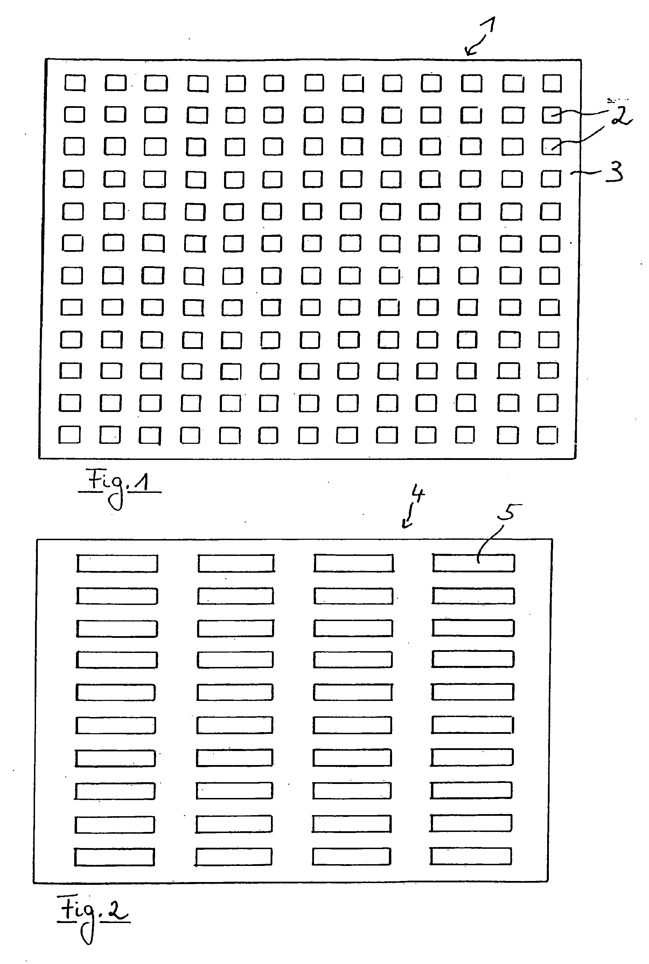

Figur 1 zeigt eine Aufsicht auf ein erfindungsgemäßes Wundkontaktmaterial 1. Das gezeigte Wundkontaktmaterial ist flächig ausgebildet und hat die Form eines Rechtecks. Neben der Rechteckform sind verschiedenste andere Formen wie Quadrate, Kreise, Trapeze usw. denkbar. Auch ist die Größe sehr variabel. Das dargestellte Wundkontaktmaterial weist Materialaussparungen 2 in Form von Löchern auf. Diese Materialaussparungen haben im vorliegenden Ausführungsbeispiel nahezu quadratische Formen. Die Materialaussparungen können jedoch auch eine andere Form, wie beispielsweise Kreisform, Dreiecksform, etc. aufweisen. Die Größe der Aussparungen ist hier zwar homogen verteilt und mit identischer Größe dargestellt, jedoch ist eine beliebige Größenverteilung und eine beliebige Aufteilung auf der Gesamtfläche des Wundkontaktmaterials möglich. Das Trägermaterial der dargestellten Ausführungsform des Wundkontaktmaterials ist als Folie ausgebildet. Das Trägermaterial kann jedoch auch als poröse Membran, Vlies, Gewebe, Gestrick, Gewirk oder Geflecht oder Kombinationen davon ausgebildet sein. Das Trägermaterial kann, abgesehen von den Materialaussparungen für Wundexsudat schlecht bis gar nicht durchlässig sein. Es ist jedoch auch möglich, dass das Trägermaterial neben den Materialaussparungen weitere, eventuell mit dem bloßen Auge nicht sichtbare, Durchtrittskanäle für das Wundexsudat aufweist. Dies wäre beispielsweise im Falle eines textilen Gewebes gegeben. Im vorliegenden Ausführungsbeispiel ist das Trägermaterial, wie in Beispiel 1 beschrieben, mit wundheilungsförderndem Material durchsetzt. Es ist jedoch auch möglich, dass das Trägermaterial mit dem wundheilungsfördernden Material beschichtet ist. Die Wirkstofffläche 3 der dargestellten Ausführungsform macht etwa 75% der Gesamtfläche des Wundkontaktmaterials aus. Ca. 25% der Fläche nehmen die Aussparungen 2 ein.

Figur 2 zeigt eine Aufsicht auf eine weitere Ausführungsform 4 des erfindungsgemäßen Wundkontaktmaterials. Auch dieses Material weist eine rechteckige Fläche auf. Ebenfalls weist diese Ausführungsform Materialaussparungen auf, die im Gegensatz zu Figur 1 rechteckige Schlitze 5 darstellen. Ansonsten gilt für diese Ausführungsform dasselbe, was für die Ausführungsform in Figur 1 gesagt wurde.

Darüberhinaus ist eine Kombination der Ausführungsformen angelehnt an Figur 1 und Figur 2 denkbar (z.B. Rechtecke und Quadrate kombiniert).

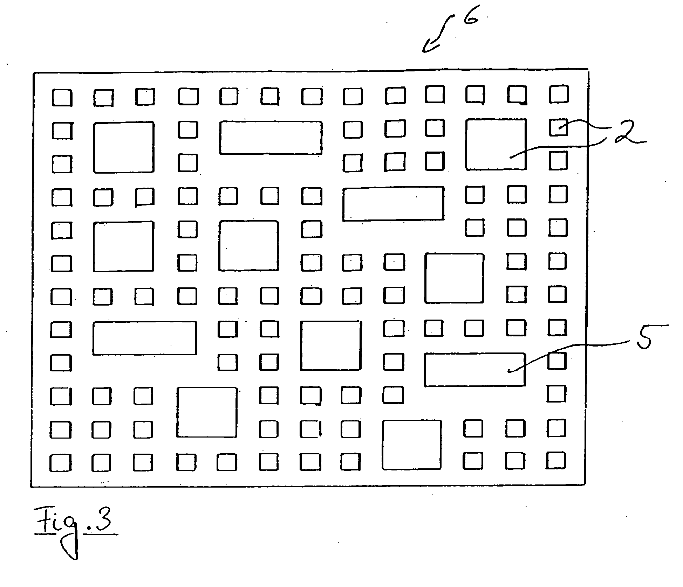

Figur 3 zeigt eine Ausführungsform 6 mit einer Kombination von verschiedenen Aussparungsformen (Rechtecke 5 und Quadrate 2) mit variabler Größenverteilung der Aussparungen. Diese Ausführungsform weist quadratische Aussparungen 2 mit zwei unterschiedlichen Größen sowie rechteckige Aussparungen 5 mit einheitlicher Größe auf. Es können jedoch auch weitere rechteckige Aussparungen unterschiedlicher Größe vorgesehen sein. Es können auch noch weitere Größen der unterschiedlichen Aussparungen vorgesehen sein. Auch können nur Aussparungen einer einzigen Form mit unterschiedlichen Größen vorgesehen sein (z.B. nur Rechtecke), wie bereits oben erwähnt.

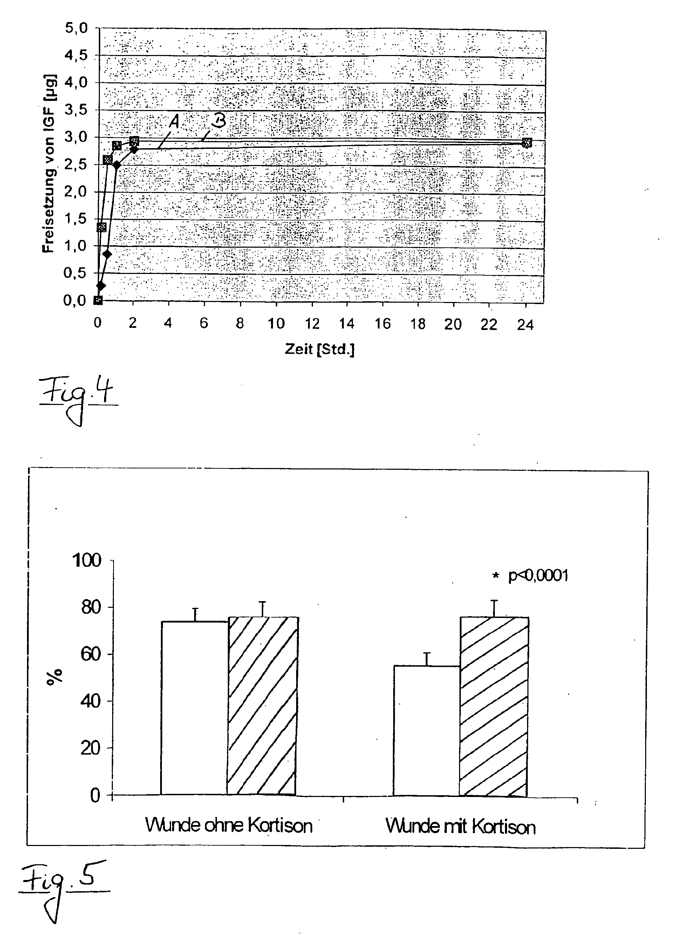

Figur 4 zeigt eine graphische Darstellung der Freisetzungskinetik am Beispiel IGF-1 aus einem erfindungsgemäßen Wundkontaktmaterial in vitro (Langzeit-Graph), wobei die Freisetzung von IGF in 0,1% BSA (Graph A) und in 4,5% BSA (Graph B) dargestellt ist. Die Initialbeladung von IGF-1 pro Verband (1 cm²) beträgt 5 µg. Aus der Figur wird ersichtlich, dass über 80% des freisetzbaren IGF innerhalb der ersten 60 Minuten freigesetzt werden. Nach ca. 24 Stunden ist das gesamte freisetzbare (60% des initialen IGF) IGF freigesetzt.

Figur 5 zeigt graphisch das Maß der Verringerungen der Wundgrößen (in %) bei IGF-Applikation mittels eines erfindungsgemäßen Wundkontaktmaterials bei nicht wundheilungsgestörten und bei wundheilungsgestörten Tieren (Ratten) (schraffierte Balken) im Vergleich zum Maß der Verringerungen der Wundgrößen bei Verwendung eines Kontrollverbandes (Wundkontakt-Verband ohne IGF) (unschraffierte Balken). Die Applikationszeit betrug jeweils 7 Tage bei täglichem Verbandswechsel und die ursprüngliche Wundgröße betrug 1 cm². Die beiden linken Balken zeigen das Maß der Verringerungen der Wundgrößen bei nicht heilungsgestörten Wunden. Die beiden rechten Balken zeigen das Maß der Verringerungen der Wundgrößen bei heilungsgestörten Wunden. Die Störung der Heilung wurde künstlich durch Kortison erzeugt (Überkortisionierung bewirkt verzögerte Wundheilung). Bei den nicht heilungsgestörten Wunden zeigten Wunden, die mit dem erfindungsgemäßen Wundkontaktmaterial mit 5µg/cm² IGF-1 behandelt wurden, eine geringfügig höhere Reduktion der Wundgröße als Wunden, die mit Standardverband behandelt wurden. Signifikant ist der Unterschied bei wundheilungsgestörten Wunden. Bei diesen zeigten jene, die mit dem erfindungsgemäßen Wundkontaktmaterial behandelt wurden, nach 7 Tagen ein Maß der Verringerungen der Wundgröße von ca. 75 % der initialen Wundgröße, während das Maß der Verringerungen der Wundgröße bei den mit dem Kontrollverband versorgten Wunden nur bei ca. 50% der initialen Wundgröße lag. Ein Vergleich der Behandlung von nicht heilungsgestörten Wunden mit heilungsgestörten Wunden zeigt, dass heilungsgestörte Wunden, die mit dem Wundkontaktmaterial nach der Erfindung behandelt wurden, ebenso gut heilen wie nicht heilungsgestörte Wunden.

### Beispiele zur Herstellung von erfindungsgemäßen Wundkontaktmaterialien:

### Beispiel 1

5g Polyvinylalkohol (PVA) werden zusammen mit 1,25g Polyethylenglycol (PEG, Molekulargewicht 2000) in 45g bidestilliertem Wasser unter Rühren auf 90°C ca. 30 bis 45 Minuten erhitzt bis eine homogene gelfreie Polymerlösung entsteht (Lösung 1). Lösung 1 wird anschließend bis zur Mischung mit Lösung 2 auf ca. 25°C abgekühlt.

### Herstellung des zellhaltigen Wundkontaktmaterials (Lösung 2):

Keratinozyten (humanen oder tierischen Ursprungs) werden nach dem Trypsinieren (2 Min. mit 0,25% Trypsin, 0,02% EDTA in PBS-Puffer) in Zentrifugenröhrchen überführt und mit serumfreien Medium 1 x gewaschen, erneut in Medium aufgezogen und ausgezählt. Nach weiteren Waschschritten und erneuter Zentrifugation wird die benötigte Zellmenge pro ml in serumfreien PBS-Puffer eingestellt. Die Zellen werden in 1ml aliquotiert. Die eingesetzte Zellzahl hängt von der Membranfläche ab, sie beträgt je nach Versuchsaufbau 20.000 bis 500.000 Zellen/cm².

In einem weiteren Schritt werden 5g von Lösung 1 und 1ml Lösung 2 zusammengegeben und ca. 30 Minuten vorsichtig verrührt. Die Mischlösung wird anschließend auf einer Rakel mit Spaltröhre 500 µm ausgestrichen. Die ausgestrichene Lösung wird bei -18°C für mindestens 4 Stunden eingefroren, dann 12 bis 16 Stunden getrocknet (< 0,1 mbar).
Die getrocknete Folie wird gemäß Figur 1 bzw. Figur 2 mechanisch ausgestanzt oder mittels Laser perforiert.
Die so präparierte, Keratinozyten-dotierte Folie (Wundkontaktmaterial) wird für einen Hydrogel/Hydrokolloid-Deckverband zugeschnitten und auf diesen gedrückt um eine innige Haftung beider Schichten zu gewährleisten. Das nach Beispiel 1 hergestellte Wundkontaktsystem kann auch mit anderen Wundverbandsmaterialien kombiniert werden.

### Beispiel 2

### Herstellung der Lösung 1

5g Polyvinylalkohol (PVA) werden zusammen mit 1,25g Polyethylenglycol (PEG, Molekulargewicht 2000) in 45g bidestilliertem Wasser unter Rühren auf 90°C ca. 30 bis 45 Minuten erhitzt bis eine homogene gelfreie Polymerlösung entsteht. Lösung 1 wird anschließend bis zur Mischung mit Lösung 2 auf ca. 25°C abgekühlt.

### Herstellung Lösung 2:

In einem Zentrifugenröhrchen werden 1 mg IGF-I (rekombinantes humanes IGF-I) in 1ml serumfreier PBS-Lösung angesetzt und vorsichtig ca. 30 Minuten lang geschüttelt, bis eine klare Lösung entstanden ist.

Anschließend werden 5g von Lösung 1 und die frisch angesetzte Lösung 2 verrührt (30 Minuten) und dann auf einer Rakel mit Spaltröhre 500 µm ausgestrichen. Die ausgestrichene Lösung wird zu einer Folie getrocknet.
Die getrocknete Folie wird gemäß Figur 1 bzw. 2 mechanisch ausgestanzt oder mittels Laser perforiert.
Das so präparierte, gemusterte IGF-dotierte Material (Wundkontaktmaterial) wird für einen Hydrogel/Hydrokolloid-Deckverband zugeschnitten und auf diesen gedrückt, um eine innige Haftung beider Schichten zu gewährleisten. Das nach Beispiel 2 hergestellte Wundkontaktsystem kann auch mit anderen Wundverbandsmaterialien kombiniert werden.

### Beispiel 3

### Lösung 1:

5g Polyvinylalkohol (PVA) werden zusammen mit 1,25g Polyethylenglycol (PEG, Molekulargewicht 2000) in 45g bidestilliertem Wasser unter Rühren auf 90°C ca. 30 bis 45 Minuten erhitzt bis eine homogene gelfreie Polymerlösung entsteht.
Die Polymerlösung wird anschließend bis zur Mischung mit Lösung 2 auf ca. 25°C abgekühlt.

### Lösung 2:

In einem Gefäß werden 1 mg IGF-I (rekombinantes humanes IGF-I) in 1ml serumfreier PBS-Lösung angesetzt und vorsichtig ca. 30 Minuten lang geschüttelt, bis eine klare Lösung entstanden ist.

Zur Herstellung einer Beschichtungslösung werden ca. 5g von Lösung 1 zusammen mit Lösung 2 verrührt. Die Viskosität der Lösung kann durch geeignete Verdickungsmittel erhöht werden. Ein textiles Flächenmaterial, wie ein Gewebe oder Vlies wird ausgebreitet und mit einem gelochten oder geschlitzten Sieb bedeckt. Durch die Sieböffnungen wird die Lösung auf das textile Material aufgetragen. Nach Abheben des Siebes wird im Vakuum getrocknet. Der so präparierte IGF-dotierte textile Verband (Wundkontaktmaterial) wird für den Hydrogel/Hydrokolloid-Deckverband zugeschnitten und auf diesen gedrückt, um eine innige Haftung beider Schichten zu gewährleisten. Das nach Beispiel 3 hergestellte Wundkontaktsystem kann aber auch mit anderen Wundverbandsmaterialien kombiniert werden. Das Aussehen des Wundkontaktsystems kann einem Negativ der Figuren 1 und 2 entsprechen, wobei die mit Wirkstoff versehenen Stellen die Form der Aussparungen 2 bzw. Schlitze 5 haben können und die übrigen miteinander verbundenen Flächen frei von Wirkstoffen sind mit entsprechend umgekehrter Flächenverteilung.

## Patentansprüche

1. Wundkontaktmaterial (1), insbesondere zur Behandlung von schlecht heilenden und chronischen Wunden wie Ulcera, mit einem flächigen Trägermaterial, welches wundheilungsförderndes freisetzbares Material aufweist, das ungleichmäßig über die Fläche des Trägermaterials verteilt ist, **dadurch gekennzeichnet, dass** das Trägermaterial hinsichtlich der Durchlässigkeit von Wundexsudat inhomogen aufgebaut ist und Stellen hoher und Stellen niedriger Durchlässigkeit für Wundexsudat aufweist, wobei das wundheilungsfördernde Material im Bereich der Stellen mit geringer Durchlässigkeit für Wundexsudat vorgesehen ist.

2. Wundkontaktmaterial nach Anspruch 1, **dadurch gekennzeichnet, dass** es im wesentlichen flexibel ausgebildet ist.

3. Wundkontaktmaterial nach Anspruch 1 oder 2, **dadurch gekennzeichnet, dass** das Trägermaterial Durchbrechungen bzw. Materialaussparungen (2) aufweist, wobei vorzugsweise die Fläche der Durchbrechungen bzw. Aussparungen 1% bis 50%, insbesondere 10% bis 30% der Gesamtfläche beträgt.

4. Wundkontaktmaterial nach Anspruch 3, **dadurch gekennzeichnet, dass** die Aussparungen bzw. Durchbrechungen hinsichtlich Größe und/oder Verteilung auf der Gesamtfläche des Wundkontakmaterials ungleichmäßig verteilt sind.

5. Wundkontaktmaterial nach einem der vorhergehenden Ansprüche, **dadurch gekennzeichnet, dass** das Trägermaterial mit dem wundheilungsfördernden Material beschichtet ist.

6. Wundkontaktmaterial nach einem der vorhergehenden Ansprüche, **dadurch gekennzeichnet, dass** das wundheilungsfördernde Material in das Trägermaterial inkorporiert ist.

7. Wundkontaktmaterial nach einem der vorhergehenden Ansprüche, **dadurch gekennzeichnet, dass** das flächige Trägermaterial als Folie, poröse Membran, Vlies, Gewebe, Gestrick, Gewirk oder Geflecht oder Kombinationen davon ausgebildet ist.

8. Wundkontaktmaterial nach einem der vorhergehenden Ansprüche, **dadurch gekennzeichnet, dass** es eine Dicke von 20 bis 200 µm besitzt und insbesondere als Folie, vorzugsweise mit einer Dicke von 20 bis 80 µm, insbesondere 30 bis 70 µm, ausgebildet ist.

9. Wundkontaktmaterial nach einem der Ansprüche 1 bis 6, **dadurch gekennzeichnet, dass** es als poröse Membran mit einer Dicke von 40 bis 200 µm, insbesondere 75 bis 150 µm, ausgebildet ist.

10. Wundkontaktmaterial nach einem der vorhergehenden Ansprüche, **dadurch gekennzeichnet, dass** das Trägermaterial aus quellfähigem und/oder wasseraufnahmefähigem Material besteht.

11. Wundkontaktmaterial nach einem der vorhergehenden Ansprüche, **dadurch gekennzeichnet, dass** das Trägermaterial aus mindestens einem Stoff aus der Gruppe bestehend aus Polyurethan, Polyvinylalkohol ,Polyethylenglycol, Silikon, Cellulose, Carboxymethylcellulose, Alginat, Polysaccharid, Chitin, Chitosan und Polyvinypyrrolidon besteht.

12. Wundkontaktmaterial nach einem der vorhergehenden Ansprüche, **dadurch gekennzeichnet, dass** das wundheilungsfördernde Material mindestens einen Wirkstoff pharmakologischen Ursprungs umfasst.

13. Wundkontaktmaterial nach einem der vorhergehenden Ansprüche, **dadurch gekennzeichnet, dass** das wundheilungsfördernde Material Kortikoide, Analgetika, Vitamine, Antibiotika, antivirale Stoffe oder Mischungen davon und/oder Hormone, insbesondere Insulin oder Zytokine, umfasst.

14. Wundkontaktmaterial nach einem der vorhergehenden Ansprüche, **dadurch gekennzeichnet, dass** das wundheilungsfördernde Material Wachstumsfaktoren, insbesondere PDGF, IGF, EGF, TGF, FGF oder Mischungen davon, umfasst.

15. Wundkontaktmaterial nach einem der vorhergehenden Ansprüche, **dadurch gekennzeichnet, dass** das wundheilungsfördernde Material menschliche und/oder tierische Zellen, insbesondere Keratinocyten, Fibroblasten, Chondrocyten, Osteoblasten oder Kombinationen davon, umfasst, wobei es vorzugsweise eine Zelldichte von etwa 20.000 bis etwa 500.000 Zellen/cm², insbesondere etwa 40.000 bis etwa 150.000 Zellen/cm², aufweist.

16. Wundkontaktmaterial nach einem der vorhergehenden Ansprüche, **dadurch gekennzeichnet, dass** es weniger als 100 µg Wirkstoff/cm² bezogen auf die Gesamtfläche, insbesondere ca. 2 bis ca. 20 µg/cm², vorzugsweise ca. 5 µg/cm², Wirkstoff enthält.

17. Wundkontaktmaterial nach einem der vorhergehenden Ansprüche, **dadurch gekennzeichnet, dass** das Trägermaterial feuchtigkeits- und flüssigkeitsdurchlässig ist und mit einer Decklage, insbesondere zur Aufnahme von Wundflüssigkeit und zur Gewährleistung eines feuchten Wundmilieus, kombiniert ist, wobei vorzugsweise die Decklage mit dem Trägermaterial auf dessen wundabgewandter Seite verbunden ist.

18. Wundkontaktmaterial nach Anspruch 17, **dadurch gekennzeichnet, dass** die Decklage von einem flüssigkeitsaufnehmbaren Material, insbesondere Hydrogel, gebildet wird.

19. Wundkontaktmaterial nach einem der vorhergehenden Ansprüche, **dadurch gekennzeichnet, dass** das Wundkontaktmaterial Stellen aufweist, die frei von wundheilungsförderndem Material sind, wobei vorzugsweise die freien Stellen mit Stellen mit erhöhter Durchlässigkeit für Wundexsudat zusammenfallen, insbesondere von Öffnungen bzw. Durchbrechungen im flächigen Trägermaterial gebildet werden.

## Claims

1. Wound contact material (1), particularly for treatment of poorly healing and chronic wounds such as ulcers, having a sheetlike backing material which includes releasable wound healing promoter material in a nonuniform distribution across the surface of the backing material, **characterized in that** the backing material has an inhomogeneous construction with regard to wound exudate permeability **in that** it includes areas of high permeability for wound exudate and areas of low permeability for exudate, the wound healing promoter material being located in the region of the areas having low permeability for wound exudate.

2. Wound contact material according to Claim 1, **characterized in that** it has an essentially flexible configuration.

3. Wound contact material according to Claim 1 or 2, **characterized in that** the backing material includes apertures or cutouts (2) and preferably the area of the apertures or cutouts comprises 1% to 50%, particularly 10% to 30% of the total area.

4. Wound contact material according to Claim 3, **characterized in that** in terms of their size and/or distribution the cutouts or apertures are distributed nonuniformly on the total area of the wound contact material.

5. Wound contact material according to any one of the preceding claims, **characterized in that** the backing material is coated with the wound healing promoter material.

6. Wound contact material according to any one of the preceding claims, **characterized in that** the wound healing promoter material is incorporated in the backing material.

7. Wound contact material according to any one of the preceding claims, **characterized in that** the sheetlike backing material is configured as foil, film, porous membrane, fibrous nonwoven web, woven fabric, knitted fabric or braided fabric or combinations thereof.

8. Wound contact material according to any one of the preceding claims, **characterized in that** it has a thickness of 20 to 200 µm and is particularly configured as foil or film, preferably having a thickness of 20 to 80 µm, particularly 30 to 70 µm.

9. Wound contact material according to any one of Claims 1 to 6, **characterized in that** it is configured as porous membrane having a thickness of 40 to 200 µm, particularly 75 to 150 µm.

10. Wound contact material according to any one of the preceding claims, **characterized in that** the backing material consists of swellable and/or water-absorbing material.

11. Wound contact material according to any one of the preceding claims, **characterized in that** the backing material consists of at least one selected from the group consisting of polyurethane, polyvinyl alcohol, polyethylene glycol, silicone, cellulose, carboxymethylcellulose, alginate, polysaccharide, chitin, chitosan and polyvinylpyrrolidone.

12. Wound contact material according to any one of the preceding claims, **characterized in that** the wound healing promoter material comprises at least one active component of pharmacological origin.

13. Wound contact material according to any one of the preceding claims, **characterized in that** the wound healing promoter material comprises corticoids, analgesics, vitamins, antibiotics, antivirals or mixtures thereof and/or hormones, particularly insulin or cytokines.

14. Wound contact material according to any one of the preceding claims, **characterized in that** the wound healing promoter material comprises growth factors, particularly PDGF, IGF, EGF, TGF, FGF or mixtures thereof.

15. Wound contact material according to any one of the preceding claims, **characterized in that** the wound healing promoter material comprises human and/or animal cells, particularly keratinocytes, fibroblasts, chondrocytes, osteoblasts or combinations thereof, and preferably has a cell density of about 20 000 to about 500 000 cells/cm², particularly about 40 000 to about 150 000 cells/cm².

16. Wound contact material according to any one of the preceding claims, **characterized in that** it contains less than 100 µg of active component/cm² based on the total area, particularly about 2 to about 20 µg/cm², preferably about 5 µg/cm², of active component.

17. Wound contact material according to any one of the preceding claims, **characterized in that** the backing material is moisture and liquid permeable and combined with a covering ply, particularly for absorbing wound fluid and for ensuring a moist wound medium, and preferably the covering ply is connected to the backing material on the wound-remote side thereof.

18. Wound contact material according to Claim 17, **characterized in that** the covering ply is formed by a fluid-absorbing material, particularly hydrogel.

19. Wound contact material according to any one of the preceding claims, **characterized in that** the wound contact material includes areas which are free of wound healing promoter material and preferably the free areas coincide with areas of enhanced permeability for wound exudate, particularly are formed by openings or apertures in the sheetlike backing material.

## Revendications

1. Matériau de contact avec une plaie (1), en particulier pour le traitement de plaies chroniques ou difficiles à guérir, telles que les ulcères, avec un substrat plan comportant un matériau libérable pour activer la guérison de la plaie, qui est réparti de façon irrégulière sur la surface du substrat, **caractérisé en ce que** le substrat est structuré de façon non homogène quant à la perméabilité à l'exsudat de la plaie, celui-ci comportant des endroits fortement perméables et des endroits faiblement perméables à l'exsudat de la plaie, le matériau activateur de guérison de plaie étant prévu aux endroits où la perméabilité à l'exsudat est plus faible.

2. Matériau de contact avec une plaie selon la revendication 1, **caractérisé en ce qu'**il est essentiellement conçu de façon élastique.

3. Matériau de contact avec une plaie selon l'une des revendications 1 ou 2, **caractérisé en ce que** le substrat comporte des interruptions ou des évidements de matériau (2), la surface de l'interruption ou de l'évidement mesurant 1 à 50% de la surface totale, de préférence 10 à 30%.

4. Matériau de contact avec une plaie selon la revendication 3, **caractérisé en ce que** les évidements ou interruptions sont répartis de façon irrégulière quant à la taille et/ou la répartition par rapport à la surface totale du matériau de contact avec une plaie.

5. Matériau de contact avec une plaie selon l'une des revendications précédentes, **caractérisé en ce que** le substrat est recouvert du matériau activateur de guérison de plaie.

6. Matériau de contact avec une plaie selon l'une des revendications précédentes, **caractérisé en ce que** le matériau activateur de guérison de plaie est incorporé au substrat.

7. Matériau de contact avec une plaie selon l'une des revendications précédentes, **caractérisé en ce que** le substrat plan est conçu comme un film, une membrane poreuse, un tissu non tissé, une toile, un maillage, un tricot ou un treillis ou encore une combinaison de ceux-ci.

8. Matériau de contact avec une plaie selon l'une des revendications précédentes, **caractérisé en ce qu'**il a une épaisseur de 20 à 200 µm et est conçu en particulier comme un film, de préférence avec une épaisseur de 20 à 80 µm, en particulier de 30 à 70 µm.

9. Matériau de contact avec une plaie selon l'une des revendications 1 à 6, **caractérisé en ce qu'**il est conçu comme une membrane poreuse, avec une épaisseur de 40 à 200 µm, en particulier de 75 à 150 µm.

10. Matériau de contact avec une plaie selon l'une des revendications précédentes, **caractérisé en ce que** le substrat est constitué d'un matériau capable de gonfler et/ou d'absorber de l'eau.

11. Matériau de contact avec une plaie selon l'une des revendications précédentes, **caractérisé en ce que** le substrat est constitué d'au moins un matériau parmi le groupe comprenant le polyuréthane, l'alcool polyvinylique, le polyéthylène glycol, la silicone, la cellulose, la cellulose carboxyméthylique, l'alginate, le polysaccharide, la chitine, le chitosan et la polyvinylpyrrolidone.

12. Matériau de contact avec une plaie selon l'une des revendications précédentes, **caractérisé en ce que** le matériau activateur de guérison de plaie comprend au moins une substance active d'origine pharmacologique.

13. Matériau de contact avec une plaie selon l'une des revendications précédentes, **caractérisé en ce que** le matériau activateur comprend des corticoïdes, des analgésiques, des vitamines, des antibiotiques, des substances antivirales ou des mélanges de ceux-ci et/ou des hormones, en particulier de l'insuline ou de la cytokine.

14. Matériau de contact avec une plaie selon l'une des revendications précédentes, **caractérisé en ce que** le matériau activateur de guérison de plaie comprend des facteurs de croissance, en particulier des PDGF, des IGF, des EGF, des TGF, des FGF ou des mélanges de ceux-ci.

15. Matériau de contact avec une plaie selon l'une des revendications précédentes, **caractérisé en ce que** le matériau activateur de guérison de plaie comprend des cellules humaines et/ou animales, en particulier des kératinocytes, des fibroblastes, des chondrocytes, des ostéoblastes ou une combinaison de ceux-ci, tout en présentant de préférence une densité de cellules d'environ 20.000 à environ 500.000 cellules/cm², en particulier d'environ 40.000 à environ 150.000 cellules/cm²_{.}

16. Matériau de contact avec une plaie selon l'une des revendications précédentes, **caractérisé en ce qu'**il contient moins de 100 µg de substance active/cm² par rapport à la surface totale, en particulier entre environ 2 et environ 20 µg/cm², de préférence environ 5 µg/cm².

17. Matériau de contact avec une plaie selon l'une des revendications précédentes, **caractérisé en ce que** le substrat est perméable à l'humidité et au liquide, tout en étant combiné avec une couche couvrante, en particulier pour l'absorption du liquide de plaie et pour garantir un milieu humide pour la plaie, la couche couvrante étant de préférence reliée au substrat de son côté détourné de la plaie.

18. Matériau de contact avec une plaie selon la revendication 17, **caractérisé en ce que** la couche couvrante est formée par une matière absorbant les liquides, en particulier de l'hydrogel.

19. Matériau de contact avec une plaie selon l'une des revendications précédentes, **caractérisé en ce que** le matériau de contact avec une plaie comporte des endroits sans matériau activateur de guérison de plaie, ces endroits correspondant de préférence à des endroits plus fortement perméables à l'exsudat de plaie, formés en particulier par des ouvertures ou des interruptions dans le substrat plan.
